(19) Europäisches Patentamt
European Patent Office
Office européen des brevets

(11) **EP 2 421 570 B1**

(12) **EUROPEAN PATENT SPECIFICATION**

(45) Date of publication and mention
of the grant of the patent:
**25.02.2015 Bulletin 2015/09**

(51) Int Cl.:
*A61L 27/56* $^{(2006.01)}$     *A61L 27/46* $^{(2006.01)}$

(21) Application number: **10726639.7**

(86) International application number:
**PCT/PL2010/050015**

(22) Date of filing: **26.04.2010**

(87) International publication number:
**WO 2010/123389 (28.10.2010 Gazette 2010/43)**

(54) **BIOACTIVE COMPOSITE AND PROCESS FOR THE PRODUCTION OF THE BIOACTIVE COMPOSITE**

BIOLOGISCH WIRKSAMER KOMPOSIT UND VERFAHREN ZUR HERSTELLUNG DES BIOLOGISCH WIRKSAMEN KOMPOSITS

COMPOSITE BIOACTIF ET PROCÉDÉ DE FABRICATION DU COMPOSITE BIOACTIF

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.04.2009 PL 38787209**

(43) Date of publication of application:
**29.02.2012 Bulletin 2012/09**

(73) Proprietor: **MEDICAL INVENTI Sp.z o.o.**
**20-262 Lublin (PL)**

(72) Inventors:
• **BELCARZ, Anna**
**20-554 Lublin (PL)**
• **GINALSKA, Grazyna**
**20-860 Lublin (PL)**
• **SLÓSARCZYK, Anna**
**32-091 Michalowice 1 (PL)**
• **PASZKIEWICZ, Zofia**
**30-658 Kraków (PL)**

(74) Representative: **Belz, Anna et al**
**Law & Patent Office Anna Belz**
**Ul. Pólnocna 6/24**
**20-064 Lublin (PL)**

(56) References cited:
**US-A- 5 017 627     US-A1- 2003 082 808**

**Description**

The present invention relates to a bioactive composite and process for production of the bioactive composite.

Ceramic materials have been used for a long time as matrices for bone defects filling in orthopaedics and maxillofacial surgery. This so called bioceramics include materials based on calcium phosphates, especially hydroxyapatites (HAp) and whitlockits (($\beta$-TCP - $\beta$-tricalcium phosphate) and also biphasic materials (BCP) composed of HAp and TCP. Other ceramic materials used as implantable ones are based on aluminium, zirconium and titanium oxides and calcium carbonate (corraline exoskeleton).

HAp bioceramics is extremely important among the modern implantable materials. It is widely applied in maxillofacial surgery, for enlargement of atrophied edge of alveolar process bone, for filling of bone defects after hemisections, radectiomies, amputations of the root of the tooth or after cysts excisions, in stomatology in treatment of deep gingival pockets, in orthopaedics for filling of bone defects resulting from trauma or neoplasms and during reconstructions of hip and knee joints. Hydroxyapatite is a preferred material for its chemical and mineralogical similarity to inorganic compounds of bone and teeth and for lack of cytotoxic and oncogenic effect on human organism. HAp bioceramics is also characterized by high osteoconductivity and biocompatibility with bone tissue and cartilage. Calcium and phosphate content enables the beneficial effect of hydroxyapatite on the process of healing and regeneration of bones. Hydroxyapatite preparations applied for medical purposes have usually porous structure and are used in a form of granules or scaffolds.

HAp bioceramics, for the mentioned features, is a good implantable material for bone defects substitution. Its porosity, in case of treatment of such defects, is a beneficial feature because - after the implantation - pores of the material are overgrown by living bone tissue, which contributes to closer biological connection between implant and bone (Lu J.X. et al., J. Mater. Sci. Mater. Res. 10, 111-120, 1999; Liu D.M., Sci. Forum 250, 183-208, 1997). An important condition of durable connection between the implant and bone tissue, obligatory for an effective placement of HAp in a defect, is an appropriate dimension of open macropores (minimum 100 $\mu$m), enabling the living tissue to grow into. Macropores contribute to osteogenesis by facilitation of osteoblasts and ions into porous granules (Bignon A. et al., J. Mater. Sci. Mater. Med. 14, 1089-97, 2003; Hulbert S.F. et al., J. Biomed. Mater. Res. 6, 347-374, 1972) or scaffolds. Moreover, the macroporous microstructure of implants is a condition for neovascularity (Henno S. et al., Biomaterials 24, 3173-3181, 2003; Freyman T.M. et al., Prog. Mater. Sci. 46, 273-282, 2001; Jones J.R. et al., J. Sol-Gel Sci. Tech. 29, 179-188, 2004). Microporosity (pores of several micrometers) - -characteristic also for HAp ceramics - also plays an important role in the implants healing, because the micropores provide attachment points for osteoblasts (Bignon A. et al., J. Mater. Sci. Mater. Med. 14, 1089-1097, 2003) and increase the surface area for protein adsorption (Hing K.A. et al., J. Mater. Sci. Mater. Med. 16, 467-475, 2005). There are reports concerning the negative effects of small particles of hydroxyapatite powder. Some authors note that particles of 1 $\mu$m in size may stimulate macrophages to secrete the proinflammatory factor - TNF$\alpha$ - thus stimulating the inflammatory process in the implantation site. Particles of 15 $\mu$m in size do not exert such a harmful effect (Nadra I. et al., Artheriosclerosis 196, 98-105, 2008).

Despite numerous advantages, HAp granules are not perfect implantable material. For their friability, they show the lack of so called surgical handiness and are difficult to handle and to maintain in the surgical sites leading to translocation within the implantation site. Mechanical parameters of HAp ceramics, especially the porous one, are also not advantageous. It concerns both granules and sintered scaffolds.. They are too brittle and not elastic enough to become a satisfactory implantable material. Young's modulus for human bone is approx. 20 GPa while for HAp material - about 5-10 times higher, which confirms low elasticity of this material.

As far, the research concerning the improvement of mentioned parameters of ceramic hydroxyapatite is limited to its modification by plasticization of hydroxyapatite powder using hydrous suspension of curdan, incubated at 40°C (United States Patent 5132255). The above method allows to obtain plastic composition which is, however, non-resistant to mechanical load. Moreover, the use of powder composed of particles of 1 $\mu$ in size does not allow to obtain the porosity in obtained composite microstructure. Modification of this method, based on firing of obtained plastic mass at temperature 800°C or higher, leads to obtaining of hard scaffolds (United States Patent 5132255). The authors of the patent do not give the data concerning the compressive strain of such prepared samples, however, in sight of such a high production temperature, it is likely that samples are deprived of fired and evaporated biopolimeric fraction, therefore they lack plasticity and elasticity. Similar method of firing leading to obtaining of ceramic scaffolds was elaborated for composition of calcium-phosphate granules with methylcellulose as binding compound, evaporating during the firing (United States Patent 6187046).

Other conception of combining the ceramic granules into compact mass concerns fibrin glue, known for its angiogenesis-stimulating properties (Takei A. et al., In Vitro Cell Dev Biol Anim 31, 467-472, 1995). Relevant experiments were performed by - among others - Nakamura et al., (Biomaterials 19, 1901-1907, 1998 who cemented the hydroxyapatite thermal decomposition product with fibrin glue and showed the osteogenic properties of such obtained composite. However, they did not give the information concerning its mechanical resistance. Similar experiments were performed by Abiraman S. et al. (Biomaterials 23, 3023-3031, 2002), who obtained the composition of fibrin glue and hydroxyapatite granules and implanted intramuscularly to mice. They did not either give the information on mechanical parameters of their composite. However, it should be noted that long-term (up to 8 years after the operation) evaluation of implantation effects in case of biomaterial composed of porous HAp granules and fibrin glue showed the good ingrowth of bone tissue into the implant structure (Fortunato G. et al., J. Cranio-Maxillofacial Surg. 25, 124-135, 1997).

Other experiments concerning the application of fibrin glue for combining the bioceramics were performed on hydroxyapatite-related calcium phosphate granules made of TCP (Le Nihouannen D. et al., Biomaterials 27, 2716-2722, 2006). It was found that such prepared composite induced the mineralization of fibrin fibrils. However, the authors do not give the information concerning the mechanical properties of obtained composite and the sole information concerns Young's overgrowth by human bone. Young's modulus from sample collected from the implants in this case was approx. 7 GPa (Le Nihouannen D. et al., J. Mater. Sci.: Mater. Med. 18, 225-235, 2007).

In case of the composite mentioned, its preparation was not so beneficial because biphasic fibrin glue required separate pre-sterilization of both compound and mixing with HAp granules before the implantation. Another disadvantage concerns the relatively long polimerization period. These factors affect the critical evaluation of created composites, especially that risk of post-operative bacterial contamination of the implant increases in such case.

American patent application US5017627A1 discloses a composite material for use in orthopaedics based on synthetic polymer. The document provides a composite of a homo- or co-polyolefin having a weight average molecular weight (Mw) greater than 20,000 with up to 80% by volume of a particulate inorganic solid. The solution covers composites showing the values of Young's modulus within the range from 2 GPa to 40 GPa. These values are typical for compact bone.

The aim of the presented invention is preparation of implantable biomaterial of high surgical handiness and optimal mechanical and biological parameters, resembling those of natural human bone.

The aim was obtained by creation of new composite formula of calcium-phosphate-polysaccharide, containing porous calcium-phosphate granules and $\beta$-1,3-glucan, further called curdlan, in appropriate proportions.

The composite related with the invention is characterized by this that it contains $\beta$-1,3-glucan, further called curdlan, and calcium-phosphate bioceramics in a form of microporous granules (HAp,. HAp-TCP, TCP, modified HAp) of size: 0.1-1.0 mm and open porosity: 50-70 %, where the compounds are in amounts (grams per 100 g of $H_2O$) according to proportion:

$$\frac{x}{y} = \frac{8{,}1 \div 25}{2 \div (0{,}0701x^2 - 5{,}8175x + 120{,}68)^{(*)}}$$

where :

    x - mass of curdlan (in g per 100 g water)
    y - mass of granules (in g per 100 g water)
    (*) - experimentally obtained formula for calculation of maximum amount of granules introduced into composite:
    $(0{,}0701x^2 - 5{,}8175x + 120{,}68)$

Curdlan, according to the invention, serves as a glue combining the granules to obtain compact composition.

The composite, prepared according to the invention, which possesses much better biological and mechanical parameters when compared with calcium-phosphate granules alone, becomes good, new generation of implantable material for bone defects filling. The prepared composite can be cut into appropriate dimensions using a lancet or a knife. The dried composite (24 hours at 40°C) is not susceptible to mould overgrowth and can be stored for at least 12 months without change in its properties. The dry composite, after soaking with liquid (water, physiological salt, gentamicin solution, protein solution) for 1-60 minutes (depending on dimensions and shape of sample) absorbs it, thus restoring its low ductile properties and can be further stored, if sterilized.

The procedure for preparation of calcium-phosphate-curdlan composite is also the subject of the invention..

The procedure, according to invention, consists in the idea that water suspension of $\beta$-1,3-glucan, further called curdlan, in amount of 8.1 $\div$ 25 g per 100 g of water, is combined with calcium-phosphate bioceramics in a form of microporous granules, in size of 0.1-1.0 mm and open porosity of 50-70% (in a range of 0.05-1.0 $\mu$m) and subjected to precise mixing, in addition to which the compounds are used in proportions described in the formula:

$$\frac{x}{y} = \frac{8.1 - 25}{2 - (0.0701x^2 - 5.8175x + 120.68)^{(*)}}$$

where:

    x - mass of curdlan (in g per 100 g water)
    y - mass of granules (in g per 100 g water)

while the most beneficial effect is when the mass of granules marked as "y" makes 95-100% of amount calculated from formula (*): $y = 0.0701x^2 - 5.8175x + 120.68$ and afterwards the mix of chosen compounds is placed in chemoresistant dishes, carefully beating down to avoid introduction of air bubbles, closed and heated for 5-30 minutes at temperature 80-100°C.

The advantage of this invention is an easy preparation procedure of composite implantable material made of calcium-phosphate and curdlan for filling of bone defects, which possesses, according to invention, advantageous value of Young's modulus and good handiness and compactness without the risk of translocation within the implantation site. Such a composite is bioactive because it contains calcium-phosphate ceramics, which - after the implantation - may release the calcium and phosphate ions into the environment, serving afterwards as a source for synthesis of native inorganic phase of the bone. The composite is also characterised by good compactness due to microporosity of ceramic phase, which enables the biopolimeric phase to migrate into the granule pores, thus binding the whole structure of the composite. The material become beneficially plastic, thanks to which it can be fit to the some extent to shape of defect and seal the site of implant-bone joint (compressive strain for composite was 20-42%). Calcium-phosphate-curdlan composite, in case of implantation, will increase the rate of natural living bone tissue regeneration. This can be performed by mechanical interactions with edges in trauma site and biostimulation of angiogenesis, cell proliferation and differentiation processes.

The following examples illustrate the invention in more detail.

Example I. Curdlan in amount of 0.625 g was mixed with 5 ml deionized water and further combined during 10 minutes with 3 g microporous HAp granules (mix of granules 0.2-0.3 mm:0.5-0.6 mm in proportion 1:3). Carefully mixed portion of the composite was placed in closed glass tubes (8 mm in diameter) and heated for 10 minutes at 95 °C. The composite was cooled down to room temperature, taken out of tubes and dried at 45 °C for 24 hours. Young's modulus for dried and soaked samples for 1 hour in physiological salt solution was approx. 0.8 GPa and 0.2 GPa, respectively. Compressive strain for these samples was approx. 23 % and 42%, respectively, and compression strength approx. 6 MPa and 0.3 MPa, respectively. The composite, in a dried form, was stored at room temperature and remained untouched by bacterial or fungal colonization.

Example II. Curdlan in amount of 0.625 g was mixed with 5 ml deionized water and further combined during 10 minutes with 3 g microporous HAp-TCP granules (fraction 0.2-0.3 mm). Carefully mixed portion of composite was placed in closed glass tubes (8 mm in diameter) and heated for 7 minutes at 95 °C. The composite was cooled down to room temperature, taken out of tubes and dried at 45°C for 24 hours. Young's modulus for dried and soaked samples for 1 hour in physiological salt solution was approx. 0.6 GPa and 0.2 GPa, respectively. Compressive strain for these samples was approx. 24 % and 32%, respectively, and compression strength approx. 5 MPa and 0.2 MPa, respectively.

Example III. Curdlan in amount of 0.625 g was mixed with 5 ml deionized water and further combined during 10 minutes with 3 g microporous TCP granules (fraction 0.3-0.4 mm). Carefully mixed portion of composite was placed in closed glass tubes (8 mm in diameter) and heated for 15 minutes at 90 °C. The composite was cooled down to room temperature, taken out of tubes and dried at 45°C for 24 hours. Young's modulus for dried and soaked samples for 1 hour in physiological salt solution was approx. 0.6 GPa and 0.2 GPa, respectively. Compressive strain for these samples was approx. 24 % and 22%, respectively, and compression strength approx. 5 MPa and 0.3 MPa, respectively.

Example IV. Curdlan in amount of 0.5 g was mixed with 5 ml deionized water and further combined during 10 minutes with 3.5 g microporous HAp granules (mix of granules 0.2-0.3 mm:0.5-0.6 mm in proportion 1:3). Carefully mixed portion of the composite was placed in closed glass tubes (12 mm in diameter) and heated for 9 minutes at 100°C. Composite was cooled down to room temperature, taken out of tubes and dried at 45°C for 24 hours. Young's modulus for s dried and soaked samples for 1 hour in physiological salt solution was approx. 0.5 GPa and 0.2 GPa, respectively. Compressive strain for these samples was approx. 23 % and 42%, respectively, and compression strength approx. 5 MPa and 0.25 MPa, respectively. Composite was subjected to sterilization by autoclaving (20 minutes at 121 °C) without change in its mechanical properties.

Example V. Curdlan in amount of 1 g was mixed with 10 ml deionized water and further combined during 10 minutes with 7 g microporous carbonated HAp granules of open porosity 60 % and average pore diameter 0.25 mm (fraction 0.2-0.3 mm). Carefully mixed portion of the composite was placed in closed glass dish (38 mm in diameter) and heated for 15 minutes at 95°C. The composite was cooled down to room temperature, taken out of dish and dried at 45°C for 24 hours. Young's modulus for dried and soaked samples for 1 hour in physiological salt solution was approx. 0.4 GPa and 0.2 GPa, respectively. Compressive strain for these samples was approx. 20 % and compression strength approx. 3 MPa and 0.15 MPa, respectively. The composite was subjected to sterilization by ethylene oxide (55 °C, 5 hours, with overnight ventilation for post-process gas removing) and it did not change in its mechanical properties.

Example VI. Curdlan in amount of 0.5 g was mixed with 5 ml deionized water and further combined during 10 minutes with 3.5 g microporous HAp granules (mix of granules 0.2-0.3 mm:0.5-0.6 mm in proportion 1:3). Carefully mixed portion of the composite was placed in closed glass tubes (10 mm in diameter) and heated for 10 minutes at 95°C. The composite was cooled down to room temperature, taken out of tubes and dried at 45°C for 24 hours. Young's modulus for dry samples was approx. 0.4 GPa. Compressive strain 23 % and compression strength approx. 4 MPa. Such obtained composite was soaked for 2 hours in physiological salt solution and retained its low-ductile properties.

Example VII. Curdlan in amount of 0.5 g was mixed with 5 ml deionized water and further combined during 10 minutes with 3.5 g microporous HAp granules (fraction 0.5-0.6 mm). Carefully mixed portion of composite was placed in closed glass tubes (8 mm in diameter) and heated for 20 minutes at 86°C. The composite was cooled down to room temperature, taken out of tubes and dried at 45°C for 24 hours. Young's modulus for dried and soaked samples for 1 hour in physiological salt solution was approx. 0.6 GPa and 0.2 GPa, respectively. Compressive strain for these samples was approx. 19 % and compression strength approx. 4 MPa and 0.2 MPa, respectively. Dried samples of the composite were soaked for 30 minutes in PBS pH 7.4 and retained its low-ductile properties.

Example VIII. Curdlan in amount of 0.81 g was mixed with 10 ml deionized water and further combined during 10 minutes with 7.81 g microporous HAp granules (fraction 0.3-0.4 mm). Carefully mixed portion of the composite was placed in closed glass tubes (8 mm in diameter) and heated for 15 minutes at 90°C. Composite was cooled down to room temperature, taken out of tubes and dried at 45°C for 24 hours. Compressive strain for dried and soaked samples for 1 hour in physiological salt solution was approx. 13 % and 20%, respectively, and compression strength approx. 3.3 MPa and 0.2 MPa, respectively

Example IX. Curdlan in amount of 0.81 g was mixed with 10 ml deionized water and further combined during 10 minutes with 3.3 g microporous HAp granules (fraction 0.3-0.4 mm). Carefully mixed portion of the composite was placed in closed glass tubes (8 mm in diameter) and heated for 10 minutes at 95°C. The composite was cooled down to room temperature, taken out of tubes and dried at 45°C for 24 hours. Compressive strain for dried and soaked samples for 1 hour in physiological salt solution was approx. 21 % and 52%, respectively, and compression strength approx. 6.7 MPa and 0.2 MPa, respectively. Dried samples of the composite were soaked for 15 minutes in PBS pH 7.4 and retained its low-ductile properties.

Example X. Curdlan in amount of 2.5 g was mixed with 10 ml deionized water and further combined during 10 minutes with 1.9 g microporous HAp granules (fraction 0.3-0.4 mm). Carefully mixed portion of the composite was placed in closed glass tubes (8 mm in diameter) and heated for 12 minutes at 95°C. The composite was cooled down to room temperature, taken out of the tubes and dried at 45°C for 24 hours. Compressive strain for dried and soaked samples for 1 hour in physiological salt solution was approx. 35 % and 47%, respectively, and compression strength approx. 27.8 MPa and 0.3 MPa, respectively. Dried samples of the composite were soaked for 2 hours in PBS pH 7.4 and retained its low-ductile properties.

## Claims

1. Bioactive composite on a base of calcium-phosphate, **characterised in that** it contains β-1,3-glucan, further called curdlan, and calcium-phosphate bioceramics in a form of microporous granules of size: 0.1-1.0 mm and open porosity: 50-70 %, where the compounds are in amounts (grams per 100 g of $H_2O$) according to proportion:

$$\frac{x}{y} = \frac{8{,}1 \div 25}{2 \div (0{,}0701x^2 - 5{,}8175x + 120{,}68)^{(*)}}$$

where :

x - mass of curdlan (in g per 100 g water)
y - mass of granules (in g per 100 g water)

**2.** The composite, according with claim 1 is **characterised in that** the mass of granules marked as "y" makes 95-100% of amount calculated from formula[*].

**3.** Procedure for preparation of calcium-phosphate-curdlan composite is **characterised in that** water suspension of $\beta$-1,3-glucan, further called curdlan, in amount of 8.1 ÷ 25 g per 100 g of water is combined with calcium-phosphate bioceramics in a form of microporous granules, in size of 0.1-1.0 mm and open porosity of 50-70%, and subjected to precise mixing, in addition to which the compounds are used in proportions described in formula:

$$\frac{x}{y} = \frac{8.1 \div 25}{2 \div (0.0701x^2 - 5.8175x + 120.68)}^{(*)}$$

where:

x - mass of curdlan (in g per 100 g water)
y - mass of granules (in g per 100 g water)

and afterwards the mix of chosen compounds is placed in chemoresistant dishes, carefully beating down to avoid introduction of air bubbles, closed and heated for 5-30 minutes at temperature 80-100°C.

**4.** The procedure according with claim 3 is **characterised in that** the mass of granules marked as "y" makes 95-100% of amount calculated from formula [*]: $y = 0.0701x^2 - 5.8175x + 120.68$.

## Patentansprüche

**1.** Das bioaktive Komposit auf Basis des Calciumphosphats, **dadurch gekennzeichnet, dass** es $\beta$-1,3- Glucan, nachstehend Curdlan genannt, sowie Biokeramik in Form von mikroporösen Granulen mit einer Größe von 0,1-1,0 mm und einer Porosität von 50-70 % enthält, wobei die Komponenten in den durch eine Proportion (Gramm pro 100 g Wasser) zusammengefassten Mengen vorkommen:

$$\frac{x}{y} = \frac{8,1 \div 25}{2 \div (0,0701x^2 - 5,8175x + 120,68)}^{(*)}$$

WO:

x - Curdlanmasse (in g pro 100 g Wasser) und
y - Granulenmasse (in g pro 100 g Wasser) kennzeichnen.

**2.** Das Komposit nach dem Anspruch 1, **dadurch gekennzeichnet, dass** die mit "y" gekennzeichnete Granulenmasse 95-100 % der sich aus der Formel ergebenden Masse beträgt (*).

**3.** Die Erzeugungsweise des bioaktiven Kompositen auf Basis der Calcium-Phosphat-Keramik, **dadurch gekennzeichnet, dass** der Wassersuspension von $\beta$-1,3- Glucan, nachstehend **Curdlan** genannt, in einer Menge von 8,1 - 25 g pro 100 g Wasser, die Calcium-Phosphat-Keramik in Form von mikroporösen Granulen mit einer Größe von 0,1-1,0 mm und einer Porosität von 50-70 % beigemengt und genau vermischt wird, wobei die Komponenten in durch eine Proportion bestimmten Mengen eingesetzt werden:

$$\frac{x}{y} = \frac{8,1 \div 25}{2 \div (0,0701x^2 - 5,8175x + 120,68)^{(*)}}$$

WO:

x - Curdlanmasse (in g pro 100 g Wasser) und
y - Granulenmasse (in g pro 100 g Wasser) kennzeichnen, wonach das Gemisch von dazu genommenen Komponenten in chemikalienfesten Formen untergebracht und zwecks Vermeidung der Einführung von Luftblasen eng gestampft, zugemacht oder zugedeckt und innerhalb von 5-30 Minuten in einer Temperatur von 80-100° C erhitzt wird.

4. Die Erzeugungsweise nach dem Anspruch 3, **dadurch gekennzeichnet, dass** die mit "y" gekennzeichnete Granulenmasse 95-100 % der sich aus der Formel ergebenden Masse beträgt (*): y = 0,0701x²-5.8175x + 120,68.

**Revendications**

1. Composé bioactif à base du phosphate de calcium **caractérisé par** la teneur en β-1-3-glucane appelé le curdlan, et par la teneur en biocéramique phosphato-calcique sous forme de granulés microporeux de dimensions 0,1 - 1,0 mm, d'une porosité de 50-70%, avec les composants présents dans les quantités (grammes pour 100g d'eau) exprimées par la proportion :

$$\frac{X}{y} = \frac{8,1 \div 25}{2 \div (0,0701x^2 - 5,8175x + 120,68)^{(*)}}$$

où

x - masse du curdlan (en g pour 100g d'eau)
y - masse des granulés (en g pour 100g d'eau)

2. Composé selon réserve 1. **caractérisé par** : la masse des granulés désignée par "Y" s'élève à 95-100% de las masse résultant de la formule (*).

3. Procédé pour fabriquer le composé bioactif à base de la céramique phosphato-calcique **caractérisé par** : à la suspension d'eau de β-1-3-glucane appelé le curdlan , dans la quantité 8,1 ÷ 25g pour 100g d'eau, on ajoute la céramique phosphato-calcique sous forme de granulés microporeux, de dimensions de 0,1 - 1,0 mm et d'une porosité de 50-70% puis on mélange bien; les composants étant utilisés dans les quantités définies par la proportion :

$$\frac{x}{y} = \frac{8,1 \div 25}{2 \div (0,0701x^2 - 5,8175x + 120,68)^{(*)}}$$

où :

x - masse du curdlan (en g pour 100g d'eau)
y - masse des granulés (en g pour 100g d'eau)

puis on place le mélange de composants assortis dans des moules à haute résistance chimique, on tasse bien pour éviter l'évacuation des bulles d'air, on ferme ou on couvre et on chauffe pendant 5-30 minutes à la température 80-100°C.

4. Procédé selon la réserve 3. **caractérisé par** : la masse des granulés désignée par "Y" s'élève à 95-100% de la masse résultant de la formule (*) : $Y = 0,0701x^2 - 5, 8175x + 120,68$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5132255 A
- US 6187046 B

- US 5017627 A1

**Non-patent literature cited in the description**

- **LU J.X. et al.** *J. Mater. Sci. Mater. Res.,* 1999, vol. 10, 111-120
- **LIU D.M.** *Sci. Forum,* 1997, vol. 250, 183-208
- **BIGNON A. et al.** *J. Mater. Sci. Mater. Med.,* 2003, vol. 14, 1089-97
- **HULBERT S.F. et al.** *J. Biomed. Mater. Res.,* 1972, vol. 6, 347-374
- **HENNO S. et al.** *Biomaterials,* 2003, vol. 24, 3173-3181
- **FREYMAN T.M. et al.** *Prog. Mater. Sci.,* 2001, vol. 46, 273-282
- **JONES J.R. et al.** *J. Sol-Gel Sci. Tech.,* 2004, vol. 29, 179-188
- **BIGNON A. et al.** *J. Mater. Sci. Mater. Med.,* 2003, vol. 14, 1089-1097

- **HING K.A. et al.** *J. Mater. Sci. Mater. Med.,* 2005, vol. 16, 467-475
- **NADRA I. et al.** *Artheriosclerosis,* 2008, vol. 196, 98-105
- **TAKEI A. et al.** *In Vitro Cell Dev Biol Anim,* 1995, vol. 31, 467-472
- **NAKAMURA et al.** *Biomaterials,* 1998, vol. 19, 1901-1907
- **ABIRAMAN S. et al.** *Biomaterials,* 2002, vol. 23, 3023-3031
- **FORTUNATO G. et al.** *J. Cranio-Maxillofacial Surg.,* 1997, vol. 25, 124-135
- **LE NIHOUANNEN D. et al.** *Biomaterials,* 2006, vol. 27, 2716-2722
- **LE NIHOUANNEN D. et al.** *J. Mater. Sci.: Mater. Med.,* 2007, vol. 18, 225-235